# EUROPEAN PATENT SPECIFICATION

(11) **EP 1 633 377 B1**
(45) Date of publication and mention of the grant of the patent: **21.03.2007**
(21) Application number: 04748592.5
(22) Date of filing: 19.05.2004
(51) Int. Cl.: A61K 33/32, A61K 33/00, A61P 3/02, A61P 17/02, A61K 31/198, A23L 1/29, A23L 1/305, A23L 1/302, A23L 1/304

(54) **A METHOD OF TREATING OR PREVENTING CHRONIC WOUNDS AND A COMPLETE NUTRITIONAL COMPOSITION COMPRISING GLYCINE AND/OR LEUCINE FOR USE THEREIN**
VERFAHREN ZUR BEHANDLUNG ODER PRÄVENTION VON CHRONISCHEN WUNDEN UND KOMPLETTE NAHRUNGSZUSAMMENSETZUNG MIT GLYCIN UND/ODER LEUCIN ZUR VERWENDUNG DARIN
METHODE PERMETTANT DE TRAITER OU DE PREVENIR DES BLESSURES CHRONIQUES ET COMPOSITION NUTRITIONNELLE COMPLETE COMPRENANT DE LA GLYCINE ET/OU DE LA LEUCINE DESTINEE A ETRE UTILISEE DANS LADITE METHODE

(30) Priority: 22.05.2003 EP 03076556
(43) Date of publication of application: 15.03.2006
(73) Proprietor: N.V. Nutricia, 2712 HM Zoetermeer (NL)
(72) Inventor: WOUTERS-WESSELING, Wendeline, Dehli, NY 13753 (US); WAGENAAR, Lisette, Wilhelmina, 3515 XK Utrecht (NL)
(74) Representative: van Westenbrugge, Andries
(86) International application number: PCT/NL2004/000355
(87) International publication number: WO 2004/103383

(56) References cited:
- EP-A- 1 228 706
- US-A- 4 780 475
- US-A- 5 576 351
- US-A- 5 656 608
- US-A- 5 922 766
- DATABASE EMBASE [Online] ELSEVIER SCIENCE PUBLISHERS, AMSTERDAM, NL; 1997, TEPASKE R: "Immunonutrition" XP002256859 Database accession no. EMB-1997165258 & CURRENT OPINION IN ANAESTHESIOLOGY 1997 UNITED KINGDOM, vol. 10, no. 2, 1997, pages 86-91, ISSN: 0952-7907
- KING P ET AL: "BRANCHED CHAIN AMINOKETO ACID SUPPLEMENTATION FOLLOWING SEVERE BURN INJURY A PRELIMINARY REPORT" CLINICAL NUTRITION (EDINBURGH), vol. 9, no. 4, 1990, pages 226-230, XP009035418 ISSN: 0261-5614

## Description

### TECHNICAL FIELD

The present invention relates to nutritional compositions for the treatment and prophylaxis of chronic wounds such as venous ulcers, diabetic ulcers and pressure sores.

### BACKGROUND OF THE INVENTION

Patients that are completely or partially immobilized for a prolonged period of time, such as hospital patients who are bedridden and patients in a wheelchair, often suffer from degenerative changes in the skin as a result of pressure and/or abrasion forces, i.e. decubitus. Decubitus patients have wounds ranging from local redness of the skin to deep wounds wherein the bone may penetrate the skin.

Wound healing is a complex and multifactorial process involving cells, extracellular matrix (ECM) components and the cellular microenvironment. Essentially all wound healing processes involve the repair or replacement of damaged tissues.
Skin comprises multiple layers, including epidermis and dermis. If only the epidermis is damaged, as in most minor injuries, epidermic cells migrate from the edge of wound and eventually cover it, repairing the epidermis. If all skin layers are damaged or destroyed, new tissue, called granulation tissue, must first fill the wound space. This tissue is formed by deposition of ECM components by fibroblasts, which migrate into the wound space. The deposition of these ECM components, such as collagen, is currently believed to be important for healing of the wound. Sufficient collagen deposition must occur to give the healing wound strength and support.

The entire multi-step process must be completed for successful wound healing, otherwise healing does not take place, the skin is not repaired and the wound remains open. Such open wounds can easily become infected, further retarding the process of healing and leading to chronic non-healing wounds. The process of wound healing may be further inhibited by complicating conditions, such as diabetes, old age, incontinence, chronic illnesses and malnutrition.

Various treatments have been proposed to accelerate the wound healing process, however, only few methods have been suggested which comprise the treatment and/or prevention of chronic wounds by the enteral administration of complete nutritional compositions containing wound healing stimulating components.

US5733884 describes a method for providing nutritional support to a patient with an acute or chronic wound comprising administering a nutritional formula containing arginine and proline.

WO9958000 describes a composition suitable for the treatment of pressure ulcers comprising carbohydrates, fats, arginine, vitamin C and tocopherol.

### SUMMARY OF THE INVENTION

The present inventors have found that presently known complete nutritional formulations are not perfectly suited for the prevention and/or treatment of chronic wounds. Hence, the present invention provides a nutritional formula, which can be advantageously used for the treatment and/or prevention of chronic wounds and that meets the unique nutrient requirements for tissue repair and healing requirements.

Patients suffering from chronic wounds were found to have a phenotypic resemblance with prolidase deficient patients, in particular the urinary excretion gly-pro dipetide. Prolidase is an enzyme that digests gly-pro dipeptide, which is an end-product of collagen breakdown. Normally, the proline and/or glycine molecules formed by lysis of gly-pro are recycled into collagen and other proteins. The inventors have unexpectedly discovered that the wound healing process can be stimulated by counteracting the effects of the impaired prolidase activity and/or by stimulating prolidase activity.

One aspect of the present invention relates to the replenishment of the glycine/proline pools through the enteral administration of a complete nutritional formulation containing significant amounts of glycine, preferably combined with significant amounts of proline and manganese. The administration of glycine and optionally proline replenishes the glycine/proline pool, thereby stimulating collagen synthesis. Manganese stimulates prolidase activity, since it is a cofactor of prolidase. Thus, administration of manganese stimulates further replenishment of the glycine/proline pool. The critical role of glycine containing nutrients in wound healing processes has not been recognized before.

In a further aspect, the present invention relates to the use of compositions high in leucine for the treatment and/or prevention of chronic wounds. It was surprisingly found that leucine is capable of stimulating protein synthesis in the wound. This was particularly unexpected as it is commonly accepted that protein synthesis in the wound is hardly affected by anabolic factors. Although some leucine is present in known nutritional compositions for patients, the present inventors found that this leucine content is insufficient for the treatment of chronic wounds. The present invention describes a method for the treatment and/or prevention of chronic wounds comprising administering to a patient a composition enriched in leucine. Moreover, it was found that compositions enriched in leucine and enriched in at least one amino acid selected from the group consisting of glycine and proline can be used particularly effectively to treat and/or prevent chronic wounds, especially decubiti ulcers.

The pathophysiology of wound healing is a complex and multifactorial process wherein a great diversity of amino acids, enzymes and cofactors play an important role. Hence, supplementation of only one or two ingredients needed in the process of wound healing will stimulate wound healing, however, the stimulatory effect will be limited since a deficiency in other amino acid, enzyme or cofactor will occur subsequently. Hence, in a particularly preferred embodiment the present composition contains a variety of ingredients that have a positive impact on wound healing processes.
It is important that, besides glycine and/or leucine, the complete nutritional composition provides sufficient energy in the form of carbohydrates and/or fats to spare protein, i.e. to prevent that protein is simply "burned". The present composition advantageously comprises a significant amount of glutamine. Glutamine is a stimulator of collagen biosynthesis in collagen producing cells and a precursor for proline and arginine. In addition, the present composition suitably comprises a significant amount of leucine. Leucine stimulates protein synthesis, which is of particular importance in patients suffering from wounds. The present composition may further contain significant amounts of arginine, since arginine was found to increase NO production and to increase collagen deposition.

Besides amino acids, many enzymes are involved in the wound healing process. Hence, in a particularly beneficial embodiment, the complete nutritional formulation contains enzyme-cofactors and other components which further stimulate the wound healing process. The present composition may suitably contain minerals such as zinc and iron. Zinc supports cell division, tissue synthesis and has a role in immunity. It has been shown that wound healing is compromised when a person is zinc deficient. Iron improves the hydroxylation of proline. Of further importance in the complete nutritional formula is ascorbic acid. Ascorbic acid catalyses the conversion of proline to hydroxyproline, a precursor of collagen, and thereby participates in tissue growth and wound healing. Also vitamin A has been reported to have profound effects on epithelial cells. However, vitamin A is preferably not administered in excessive dosages, since this may inhibit ascorbate-induced collagen synthesis.

In a further aspect, the present composition contains soluble indigestible fiber, which reduces the incidence of fecal incontinence in patients. Fecal incontinence may lead to increased humidity of the skin which increases the risk of formation of pressure sores and/or obstructs wound healing.

It was also found by the inventors that the present high protein composition, which is preferably enriched in several specific amino acids and optionally contains fibers, has a markedly low viscosity. The low viscosity is of importance since the formula is often ingested through a straw or administered through a tube.

In a further aspect the present enteric formula has a relatively low osmolality while providing the energy and nutrient requirements for a patient suffering from chronic wounds or patients susceptible to the development of chronic wounds. The low osmolality results in a reduced occurrence of diarrhea and fecal incontinence.

### DETAILED DESCRIPTION

In one aspect, the invention provides the use of leucine in the preparation of a composition for use in a method for the treatment and/or prevention of pressure ulcers, pressure sores, decubiti ulcers and/or diabetic food ulcers, said method comprising enterally administering to a patient a serving of a composition wherein protein represents between 10 and 40 % of the total caloric content of the composition; carbohydrate represents between 15 and 90 % of the total caloric content of the composition; and wherein the protein provides between 1 and 5 grams leucine per serving, said serving providing between 150 and 400 kcal.

Another aspect of the invention relates to the use of glycine in the preparation of a composition for use in a method for the treatment and/or prevention of pressure ulcers, pressure sores, decubiti ulcers and/or diabetic food ulcers, said method comprising enterally administering to a patient a serving of a composition wherein protein represents between 10 and 40 % of the total caloric content of the composition; carbohydrate represents between 15 and 90 % of the total caloric content of the composition; and wherein the protein provides between 0.5 and 5 grams glycine per serving, said serving providing between 150 and 400 kcal.

A further aspect of the invention provides a composition for enteral administration to patients wherein protein represents between 10 and 40 % of the total caloric content of the composition; fat represents between 10 and 50 % of the total caloric content of the composition; and carbohydrates represents between 15 and 80 % of the total caloric content of the composition, said composition comprising
(i) between 4 and 20 mg leucine per kcal; and
(ii) between 2 and 32 mg glycine per kcal and/or between 3 and 32 mg proline per kcal

The present invention also provides a composition for enteral administration to patients wherein protein represents between 10 and 40 % of the total caloric content of the composition; fat represents between 10 and 50 % of the total caloric content of the composition; and carbohydrates represents between 15 and 90 % of the total caloric content of the composition, said composition comprising:
(i) between 2 and 32 mg glycine per kcal;
(ii) between 3 and 32 mg proline per kcal; and
(iii) between 0.004 and 1 mg manganese per kcal.

The term "protein" as used herein refers to free amino acids and chains of amino acids linked together by covalent peptide bonds (e.g. peptides, oligopeptides and polypeptides). Preferably, the present composition comprises protein in the form of amino acids linked together by covalent peptide bonds.
The term "serving" as used herein refers to the amount which is delivered, or meant to be delivered to a subject in a single administration event. Typically, such an administration event is completed within 30 minutes, preferably within 15 minutes. A typical serving size is a full glass. By way of summary, Table I shows the preferred caloric distribution, amino acid profile and amounts of other ingredients according to preferred embodiments of the present high glycine composition. In a preferred embodiment the amino acid profile and amounts of other ingredient are expressed per serving of 250 kcal. In a further preferred embodiment the amino acid profile and amounts of other ingredient are expressed per serving of 200 ml.

**TABLE I: High glycine formulation**

| Ingredient | Preferred | More preferred | Most preferred |
|---|---|---|---|
| Glycine (gram per serving) | 0.5 - 8 | 0.75 - 5 | 0.8 - 3 |
| | | | |

| ***Caloric distribution*** | | | |
|---|---|---|---|
| Fat (en%) | 10 - 50 | 15 - 40 | 20 - 30 |
| Protein (en/%) | 10 - 40 | 20 - 40 | 25 - 35 |
| Carbohydrates (en %) | 15 - 90 | 20 - 50 | 25 - 40 |
| Caloric Density (kcal/ml) | 0.75 - 2 | 1.1 - 1.5 | 1.2 - 1.4 |
| | | | |

| ***Amino acid profile*** | | | |
|---|---|---|---|
| Proline (gram per serving) | 1.5 - 8 | 2 - 5 | 2.5- 4 |
| Arginine (gram per serving) | 0.5 - 8 | 1 - 5 | 2 - 4 |
| Glutamine (gram per serving) | 0.5 - 8 | 1 - 5 | 2.5 - 5 |
| Leucine (gram per serving) | 0.5 - 8 | 1.5 - 5 | 2.5 - 5 |
| Carnosine (gram per serving) | 0.05 - 8 | 0.1 - 5 | 0.2 - 2 |
| | | | |

| ***Mineral*** | | | |
|---|---|---|---|
| Manganese (mg per serving) | 1 - 250 | 1.5 - 100 | 3 - 50 |
| Iron (mg per serving) | 2 - 100 | 5 - 30 | 6 - 10 |
| Zinc (mg per serving) | 2-100 | 5 - 30 | 7.5 - 15 |
| | | | |

| ***Fibers*** | | | |
|---|---|---|---|
| Indigestible fiber (gram per serving) | 0.01 - 25 | 0.02 - 10 | 0.05 - 1 |
| | | | |

| ***Vitamin*** | | | |
|---|---|---|---|
| ascorbic acid (mg per serving) | 10 - 2000 | 100-1000 | 200 - 750 |
| vitamin a (mg RE per serving) | 50 - 2000 | 100-1000 | 200 - 500 |

### Amino acid profile: High glycine composition

The present glycine containing composition preferably comprises per serving at least one selected from the group consisting of:
a. between 1.5 and 8 grams, more preferably between 2 and 5 grams, even more preferably at least 2.5 gram, most preferably between 2.5 and 4 grams proline.
b. between 1.5 and 8 grams, more preferably between 1 and 5 grams, even more preferably at least 2 gram, most preferably between 2 and 4 grams arginine.
c. between 1.5 and 8 grams, more preferably between 1 and 5 grams, even more preferably at least 2.5 gram, most preferably between 2.5. and 4 grams glutamine.
d. between 1.5 and 8 grams, more preferably between 1 and 5 grams, even more preferably at least 2.5 gram, most preferably between 2.5. and 4 grams leucine; and
e. between 0.05 and 8 grams, more preferably between 0.1 and 5 grams, even more preferably at least 0.2 gram, most preferably between 0.2 and 2 grams carnosine and/or β-alanine.
Preferably, the present high glycine composition comprises per serving at least two, more preferably at least three, even more preferably all the members of the above group. Preferably, the present high glycine composition comprises at least glycine and proline, more preferably at least glycine, proline and arginine, even more preferably at least glycine, proline, arginine and glutamine, most preferably at least glycine, proline, arginine, glutamine and leucine in the amount specified above.

### Amino acid profile: High leucine composition

The present leucine containing composition preferably comprises per serving at least one selected from the group consisting of:
a. between 1.5 and 8 grams, more preferably between 2 and 5 grams, even more preferably at least 2.5 gram, most preferably between 2.5 and 4 grams proline.
b. between 1.5 and 8 grams, more preferably between 1 and 5 grams, even more preferably at least 2 gram, most preferably between 2 and 4 grams arginine.
c. between 1.5 and 8 grams, more preferably between 1 and 5 grams, even more preferably at least 2.5 gram, most preferably between 2.5. and 4 grams glutamine.
d. between 1.5 and 8 grams, more preferably between 1 and 5 grams, even more preferably at least 2.5 gram, most preferably between 2.5. and 4 grams glycine and
e. between 0.05 and 8 grams, more preferably between 0.1 and 5 grams, even more preferably at least 0.2 gram, most preferably between 0.2 and 2 grams carnosine and/or β-alanine.
Preferably the present high leucine composition comprises per serving at least two, more preferably at least three, even more preferably all the members of the above group. Preferably the present high leucine composition comprises at least glycine and/or proline, more preferably at least glycine and proline, even more preferably at least glycine, proline and arginine, most preferably at least glycine, proline, arginine, glutamine and leucine in the amount specified above.
By way of summary, Table II shows the preferred caloric distribution, amino acid profile and amounts of other ingredients of a preferred embodiment of present high leucine composition. In a preferred embodiment the amino acid profile and amounts of other ingredient are expressed per serving of 250 kcal. In a further preferred embodiment the amino acid profile and amounts of other ingredient are expressed per serving of 200 ml.

**TABLE II: High Leucine formulation**

| Ingredient | Preferred | More preferred | Most preferred |
|---|---|---|---|
| Leucine (gram per serving) | 1-7 | 1.25-5 | 1.5-3 |
| | | | |

| ***Caloric distribution*** | | | |
|---|---|---|---|
| Fat (en%) | 10 - 50 | 15 - 40 | 20-30 |
| Protein (en/%) | 10-40 | 20-40 | 25 - 35 |
| Carbohydrates (en %) | 15-90 | 20-50 1.1-1.5 | 25-40 |
| Caloric Density (kcal/ml) | 0.75-2 | | 1.2-1.4 |
| | | | |

| ***Amino acid profile*** | | | |
|---|---|---|---|
| Proline (gram per serving) | 1.5 - 8 | 2-5 | 2.5 - 4 |
| Arginine (gram per serving) | 0.5 - 8 | 1 - 5 | 2-4 |
| Glutamine (gram per serving) | 0.5 - 8 | 1-5 | 2.5-5 |
| Glycine (gram per serving) | 0.5 - 8 | 0.75 - 5 | 0.8 - 3 |
| Carnosine (gram per serving) | 0.05 - 8 | 0.1 - 5 | 0.2 - 2 |
| | | | |

| ***Mineral*** | | | |
|---|---|---|---|
| Manganese (mg per serving) | 1 - 250 | 1.5 - 100 | 3 - 50 |
| Iron (mg per serving) | 2 - 100 | 5 - 30 | 6 - 10 |
| Zinc (mg per serving) | 2 - 100 | 5 - 30 | 7.5 - 15 |
| | | | |

| ***Fibers*** | | | |
|---|---|---|---|
| Indigestible fiber (gram per serving) | 0.01- 25 | 0.02- 10 | 0.05 - 1 |
| | | | |

| ***Vitamin*** | | | |
|---|---|---|---|
| ascorbic acid (mg per serving) | 10-2000 | 100-1000 | 200-750 |
| vitamin a (mg RE per serving) | 50-2000 | 100-1000 | 200-500 |

### Caloric distribution

The protein in the present composition preferably represents between 10 and 40% of the total caloric content of the composition, more preferably between 20 and 40%, even more preferably at least 25%, most preferably between 25 and 35%.
The present composition preferably comprises casein and or whey protein.
The fat in the present composition preferably represents between 10 and 50% of the total caloric content of the composition, more preferably between 15 and 40%, even more preferably at least 20%, most preferably between 20 and 30%.
The carbohydrate in the present composition preferably represents between 15 and 90%, preferably between 15 and 80% of the total caloric content of the composition; more preferably at least 20 %, even more preferably between 20 and 50%, most preferably between 25 and 40%.
The present composition preferably has a caloric density between 0.75 and 2.5 kcal/ml, more preferably at least 1 kcal/ml, even more preferably between 1.1 and 1.5 kcal/ml, most preferably between 1.2 and 1.4 kcal/ml.
The term caloric content as used in the present invention refers to the calories provided by the nutritional ingredients and utilizable by the human body. Thus, the caloric content of indigestible fibers is at best very low.

### Fibers

A further aspect of the present invention describes a method for the prevention or treatment of chronic wounds such as pressure ulcers, said method comprising administering soluble indigestible (or sparingly digestible) fibers to a patient. The soluble fiber reduces the incidence and severity of fecal incontinence and/or diarrhea. The reduced fecal incontinence and diarrhea subsequently results in a reduced humidity of the skin and reduced bacterial concentrations in the proximity of the wound. Hence, occurrence and infections of wounds are reduced.

Preferably the present composition comprises per serving between 0.01 and 25 gram, more preferably between 0.02 and 15 grams, even more preferably at least 1 gram, most preferably between 1 and 15 grams soluble indigestible fiber.

### Cofactors

The present nutritional formula preferably contains enzyme cofactors and other components which further stimulate the wound healing process. In a preferred embodiment the present high glycine formulation contains per serving between 1 mg and 250 mg, preferably between 1.5 and 100 mg manganese, even more preferably at least 2 mg, most preferably between 3 and 50 mg manganese.
Zinc supports cell division, tissue synthesis and has a role in immunity and is preferably incorporated in an amount equivalent to between 2 mg and 100 mg, more preferably at least 5 mg, even more preferably between 5 and 30 m g, most preferably between 7.5. and 15 mg zinc per serving.
Iron improves the hydroxylation of proline. Hence, iron is preferably incorporated in an amount equivalent to between 2 mg and 100 mg, more preferably at least 5 mg, even more preferably between 5 and 30 mg, most preferably between 6 and 10 mg iron per serving.
Of further particular importance in the present complete nutritional formula is ascorbic acid. Ascorbic acid catalyses the conversion of proline to hydroxyproline, a precursor of collagen, and thereby participates in tissue growth and wound healing. The term ascorbic acid, as practiced in the present invention, includes vitamin C (ascorbic acid) and ascorbic acid equivalents, which are compounds that can be transformed to ascorbic acid in the body, such as dehydroascorbic acid, ascorbyl palmitate and other ascorbyl esters. Preferably the present composition contains between 10 and 2000 mg ascorbic acid, more preferably between 30 and 1000 mg, even more preferably at least 200 mg, most preferably between 200 and 750 mg ascorbic acid per serving.
Also vitamin A has been reported to have profound effects on epithelial cells. Hence, preferably the present composition contains between 50 and 2000 mcg Retinol equivalents (RE) vitamin A, more preferably between 100 and 1000 mcg RE, most preferably between 200 and 500 mcg RE.

### Treatment and prevention

The present invention describes a method for the treatment and prevention of chronic wounds, particularly to the treatment and/or prevention of pressure ulcers, venous leg ulcers, diabetic foot ulcers and burn injuries. The composition may also be advantageously used in patients with impaired wound healing resulting from the use of chemotherapeutic agents. In a particularly preferred embodiment, the present method describes the treatment of patients suffering with a chronic illness, even more preferably patients suffering from chronic wounds and/or patients susceptible to the development of chronic wounds, especially patients suffering from pressure ulcer, pressure sores, decubiti ulcer and/or diabetic food ulcer. The present invention particularly describes a method for stimulating wound healing. Furthermore, the present invention describes a method for preventing or treating a decrease in protein and/or collagen synthesis in the skin, particularly in damaged skin and for stimulating protein and/or collagen synthesis in the skin, particularly the damaged skin.

### Viscosity

It is desirable that the present composition exhibits a low viscosity at ambient conditions since the composition is mostly ingested through a straw or enterally administered through a tube. Low viscosity will facilitate easy administration. The viscosity can be suitably decreased by incorporation of at least a portion of the protein as amino acid, di-, or tripeptide. Hence, preferably the protein fraction comprises at least 20 wt.% low molecular protein selected from the group consisting of amino acid, di-, and/or tripeptide based on the dry weight of the protein fraction, more preferably at least 40 wt.%, even more preferably at least 60 wt.%. More preferably the protein fraction comprises at least 10 wt.% amino acid based on the total dry weight of the protein fraction, even more preferably at least 25 wt.%, most preferably at least 40 wt.%.
Preferably at least 10%, more preferably at least 25% and most preferably at least 40% of the glycine in the present composition is provided as amino acid, di-, or tripeptide, preferably as amino acid. Similarly, also proline, arginine, glutamine and leucine are provided as low molecular components in the present composition.

The present complete nutritional formulation preferably has a limited viscosity. Hence, according to a preferred embodiment the present composition has a viscosity below 50 mPas at a shear rate of 100s⁻¹ and 20°C, more preferably below 25 mPas at a shear rate of 100s⁻¹ and 20°C, even more preferably below 20 mPas at a shear rate of 100s⁻¹ and 20°C, most preferably below 15 mPas at a shear rate of 100s⁻¹ at 20°C.

Whenever the term viscosity is used in the present document, this refers to the physical parameter which can be suitably determined using a Carri-Med CSL rheometer. The used geometry is of conical shape (6 cm 2 deg acrylic cone) and the gap between plate and geometry is set on 55 µm. A linear continuous ramp shear rate is used from 0 to 150 s⁻¹ in 20 seconds. The rheometer's thermostat is set on the appropriate temperature (e.g. 20°C).

### Osmolality

In a further aspect, the occurrence and incidence of chronic wounds can be reduced by administration of a enteric formula with a low osmolality. High osmolality increases diarrhea and fecal incontinence. Hence, preferably the present formulation has an osmolality below 800 mOsm/kg, preferably below 700 mOsm/kg, even more preferably below 650 mOsm/kg, most preferably below 600 mOsm/kg.

### Serving

A serving of the present composition preferably has a volume of between 50 and 1100 ml, more preferably a volume of between 100 and 500 ml, even more preferably a volume of between 150 and 400 ml, most preferably between 150 and 300 ml. Preferably the volume administered to the patient is within the above limits. The patient may have difficulty ingesting the composition when administered in a very concentrated form or in high volume. One serving of the present composition preferably provides between 150 and 400 kcal, more preferably between 175 and 325 kcal, even more preferably at least 200 kcal, even more preferably between 225 and 275 kcal, most preferably 250 kcal. Preferably, the quantity of calories administered to the patient per serving is within the above limits as otherwise the patient may become overweight which may stimulate the formation of chronic wounds.
In a particularly preferred embodiment, the present composition is packaged in serving lots.
As will be immediately apparent from the above, the present composition can also be adequately defined by its content in amino acids and other ingredients per kcal. The preferred embodiments can be simply calculated from the above given values, e.g. the present composition preferably comprises between 4 and 20 mg leucine per kcal (between 1 and 5 grams per 250 kcal); between 2 and 32 mg glycine per kcal, and/or between 3 and 32 mg proline per kcal (1.5 g and 8 grams proline per 250 kcal).

### Concentrations

The present invention also relates to a complete nutritional composition in liquid form that contains a high concentration of glycine. These compositions can be suitably used in a method for the treatment and/or prevention of chronic wounds. The preferred concentrations of glycine and/or leucine, optionally combined with other beneficial ingredients can be calculated from the amount administered per serving as indicated above by dividing said amount by the most preferred volume of one serving, i.e. 200 ml.
In one embodiment the present invention provides a liquid composition wherein protein represents between 10 and 40 % of the total caloric content of the composition; carbohydrate represents between 15 and 90 % of the total caloric content of the composition, and which comprises between 2.5 and 40 grams glycine per liter, preferably between 3.75 and 25 grams glycine per liter, most preferably between 4 and 12 grams glycine per liter (corresponding to between 0.5 and 8 grams, between 0.75 and 5 grams, and between 0.8 and 3 grams glycine per 0.2 liter respectively).
Similarly, the other weights per serving as depicted in Table I and described herein above can be converted to the appropriate concentration ranges. Preferably, the present composition fulfills both the weight per serving requirements and the concentration requirements according to the present invention.

### Reconstitutable preparations

In even a further aspect, the present invention relates to reconstitutable preparations, preferably reconstitutable powders, which, when mixed with a predetermined volume of liquid yield a liquid nutritional product as described herein above, e.g. a liquid nutritional product protein represents between 10 and 40 % of the total caloric content of the composition; carbohydrate represents between 15 and 90 % of the total caloric content of the composition and fat represents representing between 10 and 50 % of the total caloric content of the composition and between 0.5 and 8 grams glycine per serving and/or between 2 and 32 grams glycine per liter. A serving of the present liquid nutritional product is preferably prepared from the reconstitutable preparation by the process of admixing a predetermined amount of reconstitutable powder with a predetermined volume of liquid, preferably (sterilized) water. The predetermined volume of liquid is preferably between 100 and 1000 ml, more preferably between 150 and 500 ml, even more preferably between 175 and 300 ml.

### EXAMPLES

### Example 1: Liquid enteral formulation for stimulating wound healing (one serving equals 200 ml):

An aqueous enteral formulation with a caloric content of 250 kcal was prepared from the following ingredients:
Proteinaceous matter 25% of the total calories (15.6 gram);
   caseinate; glycine-glutamine dipeptide, free amino acid proline; free amino acid arginine; free amino acid leucin and carnosine providing:
   0.9 grams glycine (4.5 grams per liter)
   2 grams proline (10 grams per liter)
   3 grams arginine (15 grams per liter)
   3 grams leucine (15 grams per liter)
   1 gram carnosine (5 grams per liter)
Carbohydrate 45% of the total calories (28 gram);
   Corn Syrup Solids, Sucrose
Fat 30% of the total calories (8.6 grams);
   Corn Oil 14% by wt.; Canola Oil 30% by wt.; MCT (Coconut Oil) 50% by wt.; Soy Lecithin 6% by wt.)
Minerals:
   Na 100mg, K 300mg, Cl 160mg, Ca 450mg, P 364mg, Mg 84mg, Fe 6mg, Zn 9mg, Cu 1350µg, Mn 2.5mg, F 0.38mg, Mo 38µg, Se 64µg, Cr 26µg, I 50µg,
Vitamins:
   Vit. A 238µg-RE; Carotenoids: 1.5mg; Vit. D 2.6µg, Vit. E 37.6mg alfa-TE, Vit. K 20 mg, Vit. B1 0.56mg, Vit. B2 1.26mg, Niacin 6.8mg NE, Pantothenic acid 2.0mg, Vit. B6 1.3mg, Folic acid 200µg, Vit. B12 1.6µg, Biotine 15µg, Vit C 250mg,
Other:
   Choline 138mg.
Osmolality: 500 mOsm (estimated)
Caloric Density: 1.0 kcal/ml

### Example 2: Effects of leucine, proline and glycine on wound healing

### Introduction.

A rabbit ear model was used to quantify the protein kinetics in the epidermis, dermis as well as in the whole skin following administration of different enteral diets. The fractional synthesis rate (FSR) and fractional breakdown rate (FBR) were determined by using stable isotopes in the method as described [Zhang, X.J., et al., Metabolism of skin and muscle protein is regulated differently in response to nutrition. Am J Physiol, 1998. **274**(3 Pt 1): p. E484-92]. The synthesis rate is a measure for the rate of protein synthesis in the wound area and the breakdown rate is a measure for the protein degradation. During wound healing the synthesis protein is higher than the rate of degradation.

### Method

One year old New Zeeland white male rabbits, weighing 9-10 pounds, housed individually and fed with Lab Chow HF #5326 (Purina Mill Inc., St. louis, MO) for weight maintenance were used in this study. On day 1, a partial thickness skin donor wound was created on the back and catheters were placed in the carotid artery and jugular vein under general anaesthesia. The animals had free access to regular rabbit chow and water for the first 3 days after injury. At 5:00 PM on day 3, a feeding tube was placed into the stomach under general anaesthesia and the animals were fasted overnight. On day 4 at 8:00 am, the rabbits were divided in 4 groups and each group thereafter received a different enteral diet (see below). Group 1 received a high proline, high glycine enteral diet (PG); Group 2 received a high proline, high leucine enteral diet (PL); Group 3 received a high proline, high glycine, high leucine diet (PGL); and Group 4 received a Control diet (Con). The composition of the PL, PG, PGL and control diet is presented below. The diets of groups 1-4 only differed in the protein composition of the enteral diet (see Table 1). The added amino acids were isonitrogeneously compensated in the casein fraction, i.e. each gram of added amino acid was compensated for by a decreased amount of casein.
At the same time isotope tracers were infused into the venous line for 8 hours during which the testing solutions were constantly infused into the feeding tube (see Zhang, X.J., et al,. Am J Physiol, 1998. **274**(3 Pt 1): p. E484-92). Blood was collected from the arterial line every hour. At the 8^{th} hour tissue samples was taken from the wounded area under general anaesthesia. Primary data analysis were rates of protein synthesis and protein breakdown. In the secondary analysis protein synthesis and protein breakdown in compliant rabbits were be analysed. The results are depicted in Table 2. Subgroup analysis was performed in rabbits not having the following criteria: wound infection or sepsis. Multivariate and pair wise ANOVA were used to calculate statistical significance. The study was a randomised double blind placebo controlled trial, with a set up as in Zhang, X.J., et al., Am J Physiol, 1998. 274(3 Pt 1): p. E484-92.

**TABLE 1: Protein compositions of the PL, PG, PLG and Control (Con)**

| **Protein** | **PG** | **PL** | **PGL** | **Con** |
|---|---|---|---|---|
| Alanine | 350 | 270 | 270 | 270 |
| Arginine | 1500 | 1500 | 1500 | 1500 |
| Glutamic acid/Glutamine | 1430 | 1530 | 1620 | 1748 |
| Glycine | 450 | 110 | 450 | 154 |
| Leucine | 530 | 1570 | 1350 | 785 |
| Proline | 1000 | 1050 | 940 | 770 |

### Ingredients Control (Con), PL, PG and PGL.

Average composition of Control product per 100 ml:
Energy 125 kcal / 525 kJ
Protein (30 En%) = 10.0 g (casein 6.2 g; whey protein 1.5 g; arginine 1.5 g). Carbohydrates (45 En%) = 14.2 g (glucose 0.1 g, lactose 1.7 g, maltose 0.4 g, saccharose 5.0 g, polysaccharides 6.5 g, other 0.5 g). Fat (25 En%) = 3.5 g (saturated 0.4 g, mono-unsaturated 2.1 g, poly-unsaturated 1.0 g, linoleic acid 0.80 g, α-linoleic acid 0.17 g.). Minerals: Na 50 mg; K 150 mg; Cl 80 mg; Ca 225 mg; P 182 mg; Mg 42 mg; Trace elements: Fe 3.0 mg; Zn 4.5 mg; Cu 675 µg; Mn 1.25 mg; F 0.19 mg; Mo 19 µg; Se 32 µg; Cr 13 µg; I 25 µg; Vitamins: vit. A 119 µg RE; carotenoids 0.75 mg; vit. D 1.3 µg; vit. E 18.8 mg α-TE; vit. K 10.0 µg; thiamine 0.28 mg; riboflavone 0.63 mg; niacin 3.4 mg NE; pantothenic acid 1.0 mg; vit. B6 0.65 mg; folic acid 100 µg; vit. B12 0.79 µg; biotin 7.5 µg; vit. C 125 mg; Other: choline 69 mg; **Osmolarity** 500 mOsmol/L.

Composition of PG, PL and PGL products:
Energy content and composition of these products was identical to the Control product, except that the protein component (30 En%) was composed of 7.7 g casein, 1.5 g arginine and added amino acids (proline, glycine and/or proline), wherein each gram of added amino acid was isonitrogeneously compensated for by a decreased amount of casein.

### Results

Table 2 gives the values for FSR minus FBR. The values are indicative for the regeneration kinetics of the skin. A higher value indicates an increased skin regeneration speed. The FSR-FBR value is therefore an excellent measure for predicting the wound healing capacity of enteral nutrition.

**TABLE 2. Protein kinetics: estimated means ± sem**

| **Group** | **FSR¹-FBR²** |
|---|---|
| PG | 1.84 ± 2.31 (n=6) |
| PL | 4.93 ± 2.45 (n=5) |
| PGL | 8.81 ± 2.31 (n=6) * |
| Con | 1.07 ± 2.54 (n=5) |

| | |
|---|---|
| ¹ Fractional Synthesis Rate (FSR), in %/day ² Fractional Breakdown Rate (FBR), in %/day * Significantly different from Control (p<0.1) | |

Administration of increased amounts of proline and glycine (PG); proline and leucine (PL); or proline, glycine and leucine (PGL) improved fractional synthesis rate - fractional breakdown rate (FSR-FBR). This is indicative for the advantageous impact of increased amounts of the above combinations of amino acids in a method for the treatment and/or prevention of chronic wounds, i.e. indicative for the advantageous effects of the present invention.
Compositions containing about at least 900 mg proline/100ml (e.g. 1000mg proline/100 ml) and about at least 1250 mg leucine/100 ml (e.g. about 1400 mg leucine/100 ml) were particularly effective in improving fractional synthesis rate - fractional breakdown rate. These results are indicative of the advantageous use of compositions containing at least 10 gram proline per liter; and at least 5 gram leucine per liter, preferably at least 14 gram leucine per liter; in a method for the treatment and/or prevention of chronic wounds.

A composition enriched in proline, leucine and glycine (PGL group) gave excellent results (see Table 2). Net-protein synthesis was dramatically increased in the PGL group compared with the standard nutritional formula (Con). This result is indicative of the advantageous impact of compositions containing at least 1 gram glycine per liter, preferably at least 4.5 gram glycine per liter; at least 10 gram proline per liter; and at least 5 gram leucine per liter, preferably at least 14 gram leucine per liter; in a method for the treatment and/or prevention of chronic wounds.

## Claims

1. Use of leucine in the preparation of a composition for use in a method for the treatment and/or prevention of pressure ulcers, pressure sores, decubiti ulcers and/or diabetic food ulcers, said method comprising enterally administering to a patient a serving of a composition wherein protein represents between 10 and 40 % of the total caloric content of the composition; carbohydrate represents between 15 and 90 % of the total caloric content of the composition; and wherein the protein provides between 1 and 5 grams leucine per serving, said serving providing between 150 and 400 kcal.

2. Use according to any one of the preceding claims, wherein the composition further comprises between 0.5 g and 5 grams glycine per serving.

3. Use of glycine in the preparation of a composition for use in a method for the treatment and/or prevention of pressure ulcers, pressure sores, decubiti ulcers and/or diabetic food ulcers, said method comprising enterally administering to a patient a serving of a composition wherein protein represents between 10 and 40 % of the total caloric content of the composition; carbohydrate represents between 15 and 90 % of the total caloric content of the composition; and wherein the protein provides between 0.5 and 5 grams glycine per serving, said serving providing between 150 and 400 kcal.

4. Use according to any one of the preceding claims, wherein the composition further comprises between 1 and 250 mg manganese per serving.

5. Use according to any one of the preceding claims, wherein the composition further comprises fat, said fat representing between 10 and 50 % of the total caloric content of the composition.

6. Use according to any one of the preceding claims, wherein the composition further comprises between 1.5 and 8 grams proline per serving.

7. Use according to any one of the preceding claims, wherein the composition further comprises
(i) between 0.5 and 8 grams glutamine per serving, preferably between 1 and 5 grams glutamine per serving;
(ii) between 0.5 and 8 grams leucine per serving, preferably between 1.5 and 5 grams leucine per serving; and
(iii) between 0.5 and 8 grams arginine per serving, preferably between 1 and 5 grams arginine per serving.

8. Use according to any one of the preceding claims, wherein the composition further comprises between 0.05 and 5 grams of carnosine and/or β-alanine per serving, preferably between 0.1 and 2.5 grams carnosine and/or β-alanine per serving.

9. Use according to any one of the preceding claims, wherein the composition further comprises
(i) between 2 and 100 mg iron per serving;
(ii) between 2 and 100 mg zinc per serving; and
(iii) between 10 - 2000 mg ascorbic acid per serving, preferably between 100 and 1000 mg ascorbic acid per serving

10. Use according to any one of the preceding claims, wherein the composition is a liquid and has a viscosity of less than 25 mPas at a shear rate of 100s⁻¹ and 20°C, preferably a viscosity of less than 20 mPas at a shear rate of 100s⁻¹ and 20°C.

11. Use according to any one of the preceding claims, wherein the composition further comprises between 0.01 and 25 grams indigestible soluble fiber per serving.

12. Use according to any one of the preceding claims, wherein the composition has an osmolality below 800 mOsm/kg, preferably below 700 mOsm/kg.

13. A composition for enteral administration to patients wherein protein represents between 10 and 40 % of the total caloric content of the composition; fat represents between 10 and 50 % of the total caloric content of the composition; and carbohydrates represents between 15 and 80 % of the total caloric content of the composition, said composition comprising
(i) between 4 and 20 mg leucine per kcal; and
(ii) between 2 and 32 mg glycine per kcal and/or between 3 and 32 mg proline per kcal

14. A composition for enteral administration to patients wherein protein represents between 10 and 40 % of the total caloric content of the composition; fat represents between 10 and 50 % of the total caloric content of the composition; and carbohydrates represents between 15 and 90 % of the total caloric content of the composition, said composition comprising:
(i) between 2 and 32 mg glycine per kcal;
(ii) between 3 and 32 mg proline per kcal; and
(iii) between 0.004 and 1 mg manganese per kcal.

15. The composition according to claim 14, further comprising:
(i) between 2 and 32 mg arginine per kcal
(ii) between 2 and 32 mg glutamine per kcal; and
(iii) between 2 and 32 mg leucine per kcal.

16. The composition according to any one of claims 13-15, further comprising:
(i) between 0.008 and 0.4 mg iron per kcal;
(ii) between 0.008 and 0.4mg zinc per kcal; and
(iii) between 0.04 and 8 mg ascorbic acid per kcal

17. Use according to claim 1, for treating or preventing decreased protein or collagen synthesis in the skin or for stimulating protein and or collagen synthesis in the skin.

## Patentansprüche

1. Verwendung von Leucin zur Herstellung einer Zusammensetzung für die Verwendung in einem Verfahren zur Behandlung und/oder Vorbeugung von Druckgeschwüren, Dekubitus, Dekubitalulkus und/oder diabetischem Nahrungsmittelulkus, wobei das Verfahren umfasst: enterale Verabreichung einer Portion einer Zusammensetzung an einen Patienten, bei der Proteine zwischen 10 und 40 % des gesamten Kaloriengehaltes der Zusammensetzung ausmachen und Kohlenhydrate zwischen 15 und 90 % des gesamten Kaloriengehaltes der Zusammensetzung ausmachen und wobei das Protein zwischen 1 und 5 g Leucin pro Portion bereitstellt und die Portion zwischen 150 und 400 kcal bereitstellt.

2. Verwendung nach einem der vorhergehenden Ansprüche, wobei die Zusammensetzung weiter zwischen 0,5 g und 5 g Glycin pro Portion umfasst.

3. Verwendung von Glycin zur Herstellung einer Zusammensetzung für die Verwendung in einem Verfahren zur Behandlung und/oder Vorbeugung von Druckgeschwüren, Dekubitus, Dekubitalulkus und/oder diabetischem Nahrungsmittelulkus, wobei das Verfahren umfasst: enterale Verabreichung einer Portion einer Zusammensetzung an einen Patienten, bei der Proteine zwischen 10 und 40 % des gesamten Kaloriengehaltes der Zusammensetzung ausmachen und Kohlenhydrate zwischen 15 und 90 % des gesamten Kaloriengehaltes der Zusammensetzung ausmachen und wobei das Protein zwischen 0,5 und 5 g Leucin pro Portion bereitstellt und die Portion zwischen 150 und 400 kcal bereitstellt.

4. Verwendung nach einem der vorhergehenden Ansprüche, wobei die Zusammensetzung weiter zwischen 1 und 250 mg Mangan pro Portion umfasst.

5. Verwendung nach einem der vorhergehenden Ansprüche, wobei die Zusammensetzung weiter Fett umfasst, wobei das Fett zwischen 10 und 50 % des gesamten Kaloriengehaltes der Zusammensetzung ausmacht.

6. Verwendung nach einem der vorhergehenden Ansprüche, wobei die Zusammensetzung weiter zwischen 1,5 und 8 g Prolin pro Portion umfasst.

7. Verwendung nach einem der vorhergehenden Ansprüche, wobei die Zusammensetzung weiter umfasst:
(i) zwischen 0,5 und 8 g Glutamin pro Portion, vorzugsweise zwischen 1 und 5 g Glutamin pro Portion;
(ii) zwischen 0,5 und 8 g Leucin pro Portion, vorzugsweise zwischen 1,5 und 5 g Leucin pro Portion, und
(iii) zwischen 0,5 und 8 g Arginin pro Portion, vorzugsweise zwischen 1 und 5 g Arginin pro Portion.

8. Verwendung nach einem der vorhergehenden Ansprüche, wobei die Zusammensetzung weiter zwischen 0,05 und 5 g Carnosin und/oder β-Alanin pro Portion, vorzugsweise zwischen 0,1 und 2,5 g Carnosin und/oder β-Alanin pro Portion umfasst.

9. Verwendung nach einem der vorhergehenden Ansprüche, wobei die Zusammensetzung weiter umfasst:
(i) zwischen 2 und 100 mg Eisen pro Portion;
(ii) zwischen 2 und 100 mg Zink pro Portion, und
(iii) zwischen 10 - 2000 mg Ascorbinsäure pro Portion, vorzugsweise zwischen 100 und 1000 mg Ascorbinsäure pro Portion.

10. Verwendung nach einem der vorhergehenden Ansprüche, wobei die Zusammensetzung eine Flüssigkeit ist und eine Viskosität von weniger als 25 mPas bei einer Scherrate von 100 s⁻¹ und 20° C, vorzugsweise eine Viskosität von weniger als 20 mPas bei einer Scherrate von 100 s⁻¹ und 20° C aufweist.

11. Verwendung nach einem der vorhergehenden Ansprüche, wobei die Zusammensetzung weiter zwischen 0,01 und 25 g unverdaulicher löslicher Faser pro Portion umfasst.

12. Verwendung nach einem der vorhergehenden Ansprüche, wobei die Zusammensetzung eine Osmolarität von weniger als 800 mOsm/kg, vorzugsweise von weniger als 700 mOsm/kg besitzt.

13. Zusammensetzung zur enteralen Verabreichung an Patienten, wobei Proteine zwischen 10 und 40 % des gesamten Kaloriengehaltes der Zusammensetzung ausmachen, Fett zwischen 10 und 50 % des gesamten Kaloriengehaltes der Zusammensetzung ausmacht und Kohlenhydrate zwischen 15 und 80 % des gesamten Kaloriengehaltes der Zusammensetzung ausmachen, wobei die Zusammensetzung umfasst:
(i) zwischen 4 und 20 mg Leucin pro kcal, und
(ii) zwischen 2 und 32 mg Glycin pro kcal und/oder zwischen 3 und 32 mg Prolin pro kcal.

14. Zusammensetzung zur enteralen Verabreichung an Patienten, wobei Proteine zwischen 10 und 40 % des gesamten Kaloriengehaltes der Zusammensetzung ausmachen, Fett zwischen 10 und 50 % des gesamten Kaloriengehaltes der Zusammensetzung ausmacht und Kohlenhydrate zwischen 15 und 90 % des gesamten Kaloriengehaltes der Zusammensetzung ausmachen, wobei die Zusammensetzung umfasst:
(i) zwischen 2 und 32 mg Glycin pro kcal;
(ii) zwischen 3 und 32 mg Prolin pro kcal, und
(iii) zwischen 0,004 und 1 mg Mangan pro kcal.

15. Zusammensetzung nach Anspruch 14, ferner umfassend:
(i) zwischen 2 und 32 mg Arginin pro kcal;
(ii) zwischen 2 und 32 mg Glutamin pro kcal, und
(iii) zwischen 2 und 32 mg Leucin pro kcal.

16. Zusammensetzung nach einem der Ansprüche 13 bis 15, weiter umfassend:
(i) zwischen 0,008 und 0,4 mg Eisen pro kcal;
(ii) zwischen 0,008 und 0,4 mg Zink pro kcal, und
(iii) zwischen 0,04 und 8 mg Ascorbinsäure pro kcal.

17. Verwendung nach Anspruch 1, zur Behandlung oder Vorbeugung verminderter Protein- oder Kollagensynthese in der Haut oder zur Stimulation der Protein- und/oder Kollagensynthese in der Haut.

## Revendications

1. Utilisation de leucine dans la préparation d'une composition pour utilisation dans une méthode pour le traitement et/ou la prévention d'ulcères de pression, de plaies de pression, d'ulcères de décubitus et/ou d'ulcères diabétiques alimentaires, ladite méthode comprenant l'administration entérale à un patient d'une ration d'une composition dans laquelle les protéines représentent entre 10 % et 40 % de la teneur en calories totale de la composition ; les glucides représentent entre 15 % et 90 % de la teneur en calories totale de la composition ; et dans laquelle les protéines fournissent entre 1 et 5 grammes de leucine par ration, ladite ration fournissant entre 150 et 400 kcal.

2. Utilisation selon l'une quelconque des revendications précédentes, dans laquelle la composition comprend, en outre, entre 0,5 et 5 grammes de glycine par ration.

3. Utilisation de glycine dans la préparation d'une composition pour utilisation dans une méthode pour le traitement et/ou la prévention d'ulcères de pression, de plaies de pression, d'ulcères de décubitus et/ou d'ulcères diabétiques alimentaires, ladite méthode comprenant l'administration entérale à un patient d'une ration d'une composition dans laquelle les protéines représentent entre 10 % et 40 % de la teneur en calories totale de la composition ; les glucides représentent entre 15 % et 90 % de la teneur en calories totale de la composition ; et dans laquelle les protéines fournissent entre 0,5 et 5 grammes de glycine par ration, ladite ration fournissant entre 150 et 400 kcal.

4. Utilisation selon l'une quelconque des revendications précédentes, dans laquelle la composition comprend, en outre, entre 1 et 250 mg de manganèse par ration.

5. Utilisation selon l'une quelconque des revendications précédentes, dans laquelle la composition comprend, en outre, des graisses, lesdites graisses représentant entre 10 % et 50 % de la teneur en calories totale de la composition.

6. Utilisation selon l'une quelconque des revendications précédentes, dans laquelle la composition comprend, en outre, entre 1,5 et 8 grammes de proline par ration.

7. Utilisation selon l'une quelconque des revendications précédentes, dans laquelle la composition comprend, en outre :
(i) entre 0,5 et 8 grammes de glutamine par ration, de préférence entre 1 et 5 grammes de glutamine par ration ;
(ii) entre 0,5 et 8 grammes de leucine par ration, de préférence entre 1,5 et 5 grammes de leucine par ration ; et
(iii) entre 0,5 et 8 grammes d'arginine par ration, de préférence entre 1 et 5 grammes d'arginine par ration.

8. Utilisation selon l'une quelconque des revendications précédentes, dans laquelle la composition comprend, en outre, entre 0,05 et 5 grammes de carnosine et/ou de β-alanine par ration, de préférence entre 0,1 et 2,5 grammes de carnosine et/ou de β-alanine par ration.

9. Utilisation selon l'une quelconque des revendications précédentes, dans laquelle la composition comprend, en outre :
(i) entre 2 et 100 mg de fer par ration ;
(ii) entre 2 et 100 mg de zinc par ration ; et
(iii) entre 10 et 2 000 mg d'acide ascorbique par ration, de préférence entre 100 et 1 000 mg d'acide ascorbique par ration.

10. Utilisation selon l'une quelconque des revendications précédentes, dans laquelle la composition est un liquide et a une viscosité inférieure à 25 mPas à un taux de cisaillement de 100s⁻¹ et 20°C, de préférence une viscosité inférieure à 20 mPas à un taux de cisaillement de 100s⁻¹ et 20°C.

11. Utilisation selon l'une quelconque des revendications précédentes, dans laquelle la composition comprend, en outre, entre 0,01 et 25 grammes de fibre soluble indigestible par ration.

12. Utilisation selon l'une quelconque des revendications précédentes, dans laquelle la composition a une osmolalité inférieure à 800 mOsm/kg, de préférence inférieure à 700 mOsm/kg.

13. Composition pour administration entérale à des patients, dans laquelle les protéines représentent entre 10 % et 40 % de la teneur en calories totale de la composition ; les graisses représentent entre 10 % et 50 % de la teneur en calories totale de la composition ; et les glucides représentent entre 15 % et 80 % de la teneur en calories totale de la composition, ladite composition comprenant :
(i) entre 4 et 20 mg de leucine par kcal ; et
(ii) entre 2 et 32 mg de glycine par kcal et/ou entre 3 et 32 mg de proline par kcal.

14. Composition pour administration entérale à des patients, dans laquelle les protéines représentent entre 10 % et 40 % de la teneur en calories totale de la composition ; les graisses représentent entre 10 % et 50 % de la teneur en calories totale de la composition ; et les glucides représentent entre 15 % et 90 % de la teneur en calories totale de la composition, ladite composition comprenant :
(i) entre 2 et 32 mg de glycine par kcal ;
(ii) entre 3 et 32 mg de proline par kcal ; et
(iii) entre 0,004 et 1 mg de manganèse par kcal.

15. Composition selon la revendication 14, comprenant en outre :
(i) entre 2 et 32 mg d'arginine par kcal ;
(ii) entre 2 et 32 mg de glutamine par kcal ; et
(iii) entre 2 et 32 mg de leucine par kcal.

16. Composition selon l'une quelconque des revendications 13 à 15, comprenant en outre :
(i) entre 0,008 et 0,4 mg de fer par kcal ;
(ii) entre 0,008 et 0,4 mg de zinc par kcal ; et
(iii) entre 0,04 et 8 mg d'acide ascorbique par kcal.

17. Utilisation selon la revendication 1, pour empêcher ou traiter la diminution de la synthèse du collagène ou des protéines dans la peau ou pour stimuler la synthèse du collagène ou des protéines dans la peau.
